# EUROPEAN PATENT APPLICATION

(11) **EP 4 353 245 A1**
(43) Date of publication of application: **17.04.2024**
(21) Application number: 22820480.6
(22) Date of filing: 02.06.2022
(51) Int. Cl.: A61K 35/744, A61P 29/00, A61P 35/00, A23L 33/135

(54) **COMPOSITION FOR PREVENTING OR TREATING INFLAMMATORY DISEASES OR CANCER, COMPRISING LEUCONOSTOC BACTERIA-DERIVED VESICLES**

(30) Priority: 07.06.2021 KR 20210073174
(71) Applicant: MD Healthcare Inc., Seoul 03923 (KR)
(72) Inventor: KIM, Yoon-Keun, Paju-Si, Gyeonggi-do 10908 (KR)
(74) Representative: Dehns
(86) International application number: PCT/KR2022/007866
(87) International publication number: WO 2022/260352

(57) **Abstract**

The present invention relates to *Leuconostoc* bacteria-derived vesicles and a use thereof, and more particularly, to a composition for alleviating, preventing, or treating inflammatory diseases or cancer, including *Leuconostoc* bacteria-derived vesicles, which are able to effectively prevent or treat inflammatory diseases and cancer development and progression, as an active ingredient.

## Description

### [Technical Field]

The present invention relates to *Leuconostoc* bacteria-derived vesicles and a use thereof, and more particularly, to a composition for preventing or treating an inflammatory disease or cancer, comprising *Leuconostoc* bacteria-derived vesicles as an active ingredient.

This application claims priority to and the benefit of Korean Patent Application Nos. 10-2021-0073174 and 10-2022-0067757 filed in the Korean Intellectual Property Office on June 7, 2021 and June 2, 2022, respectively, and all the contents disclosed in the specification and drawings of the applications are incorporated in this application.

### [Background Art]

As we enter the 21^{st} century, the significance of acute infectious diseases, previously recognized as contagious diseases, has diminished, whereas the disease pattern has changed such that chronic diseases, accompanied by immune dysfunction due to a mismatch between humans and microbiomes, have become the major diseases that determine the quality of life and human lifespan. The chronic diseases are characterized by chronic inflammation accompanied by immune dysfunction, and various chronic inflammatory diseases and cancers caused thereby are becoming a major public health problem.

Meanwhile, it is known that the number of microorganisms that coexist in the human body reaches 100 trillion, which is about 10-fold larger than that of human cells, and the number of genes of microorganisms is 100-fold larger than that of humans. A microbiota or microbiome refers to a microbial community including bacteria, archaea and eukarya present in a given habitat.

Bacteria that coexist in our bodies and bacteria that exist in the surrounding environment secrete nanometer-sized vesicles to exchange information such as genes, low molecular compounds, and proteins with other cells. The mucosa forms a physical defense membrane through which particles having a size of 200 nanometers (nm) or more cannot pass, so that bacteria coexisting in the mucosa cannot pass through the mucosa, but bacteria-derived vesicles have a size of 200 nanometers or less, and thus relatively freely pass through epithelial cells via the mucosa to be absorbed in our bodies. As described above, although bacteria-derived vesicles are secreted from bacteria, they differ from bacteria in terms of their constituents, absorption rate in the body, and risk of side effects, and therefore, the use of bacteria-derived vesicles is completely different from that of living cells or has a significant effect.

Locally secreted bacteria-derived vesicles are not only absorbed through epithelial cells of the mucous membrane and induce a local inflammatory response, but also vesicles passing through the epithelial cells are absorbed systemically through lymphatic vessels and distributed to various organs where the vesicles regulate metabolic and immune functions. For example, pathogenic nanoparticles, which are vesicles derived from pathogenic gram-negative bacteria such as *Escherichia coli*, locally cause colitis or food poisoning. When the vesicles are absorbed into blood vessels, they are absorbed into vascular endothelial cells and induce an inflammatory response, promoting a systemic inflammatory response and blood coagulation. Furthermore, the vesicles are absorbed into muscle cells where insulin acts, triggering metabolic diseases such as insulin resistance and diabetes. On the other hand, vesicles derived from beneficial bacteria can regulate diseases by regulating immune and metabolic dysfunctions caused by pathogenic vesicles.

*Leuconostoc* bacteria are gram positive bacteria that are isolated from fermented food such as kimchi. *Leuconostoc* bacteria include *Leuconostoc carnosum*, *Leuconostoc citreum*, *Leuconostoc fallax*, *Leuconostoc falkenbergense*, *Leuconostoc ficulneum*, *Leuconostoc fructosum*, *Leuconostoc garlicum*, *Leuconostoc gasicomitatum*, *Leuconostoc gelidum*, *Leuconostoc inhae*, *Leuconostoc kimchii*, *Leuconostoc lactis*, *Leuconostoc mesenteroides*, *Leuconostoc miyukkimchii*, *Leuconostoc palmae*, *Leuconostoc pseudoficulneum*, *Leuconostoc pseudomesenteroides*, *Leuconostoc rapi*, and *Leuconostoc suionicum.*

However, it has not been reported that *Leuconostoc* bacteria extracellularly secrete vesicles, and particularly, there have been no cases of applying *Leuconostoc* bacteria-derived vesicles for preventing or treating inflammatory diseases or cancer.

### [Disclosure]

### [Technical Problem]

The present invention was invented to solve the above problems of the related art, and is directed to providing a composition for alleviating, preventing or treating inflammatory diseases or cancer, comprising *Leuconostoc* bacteria-derived vesicles as an active ingredient.

However, technical problems to be solved in the present invention are not limited to the above-described problems, and other problems which are not described herein will be fully understood by those of ordinary skill in the art from the following descriptions.

### [Technical Solution]

To achieve the purpose of the present invention, the present invention provides a pharmaceutical composition for preventing or treating inflammatory diseases or cancer, comprising *Leuconostoc* bacteria-derived vesicles as an active ingredient.

In addition, the present invention provides a food composition for preventing or alleviating inflammatory diseases or cancer, comprising *Leuconostoc* bacteria-derived vesicles as an active ingredient.

In one embodiment of the present invention, the inflammatory diseases may comprise the following diseases, but the present invention is not limited thereto:
one or more respiratory inflammatory diseases selected from the group consisting of asthma, chronic obstructive pulmonary disease, pneumonia, interstitial pneumonia, idiopathic pulmonary fibrosis, and rhinitis;
one or more skin inflammatory diseases selected from the group consisting of atopic dermatitis, psoriasis, acne, contact dermatitis, and hair loss;
one or more digestive inflammatory diseases selected from the group consisting of gastritis, digestive ulcers, acute enteritis, chronic enteritis, Crohn's disease, inflammatory bowel disease, Behcet's colitis, ulcerative colitis, bacterial colitis, alcoholic steatohepatitis, non-alcoholic steatohepatitis, chronic hepatitis, pancreatitis, and cholangitis;
vaginitis; and
one or more bone and joint inflammatory diseases selected from the group consisting of osteoarthritis, degenerative arthritis, and rheumatoid arthritis.

In another embodiment of the present invention, the inflammatory diseases may be diseases mediated by IL-6, but the present invention is not limited thereto, and may comprise any diseases caused by an inflammatory response.

In still another embodiment of the present invention, the cancer may be one or more selected from the group consisting of colon cancer, stomach cancer, lung cancer, liver cancer, biliary tract cancer, pancreatic cancer, breast cancer, ovarian cancer, kidney cancer, bladder cancer, prostate cancer, blood cancer, head and neck cancer, colon-rectal cancer, bone marrow cancer, uterine cancer, melanoma, brain cancer, thyroid cancer, and lymphoma, but the present invention is not limited thereto.

In yet another embodiment of the present invention, the *Leuconostoc* bacteria may be one or more selected from the group consisting of *Leuconostoc carnosum*, *Leuconostoc citreum*, *Leuconostoc fallax*, *Leuconostoc falkenbergense*, *Leuconostoc ficulneum*, *Leuconostoc fructosum*, *Leuconostoc garlicum*, *Leuconostoc gasicomitatum*, *Leuconostoc gelidum*, *Leuconostoc inhae*, *Leuconostoc kimchii*, *Leuconostoc lactis*, *Leuconostoc mesenteroides*, *Leuconostoc miyukkimchii*, *Leuconostoc palmae*, *Leuconostoc pseudoficulneum*, *Leuconostoc pseudomesenteroides*, *Leuconostoc rapi*, and *Leuconostoc suionicum*, but the present invention is not limited thereto.

As another exemplary embodiment of the present invention, the vesicles may have an average diameter of 10 to 300 nm, but the present invention is not limited thereto.

In yet another embodiment of the present invention, the vesicles may be naturally secreted or artificially produced from *Leuconostoc* bacteria, but the present invention is not limited thereto, and may comrpise any vesicles that are isolated from *Leuconostoc* bacteria without limitation.

In yet another embodiment of the present invention, the vesicles may be isolated from the culture medium of *Leuconostoc* bacteria or food that has been prepared by adding *Leuconostoc* bacteria, but the present invention is not limited thereto.

In addition, the present invention provides a method for preventing or treating inflammatory diseases or cancer, the method comprising administering a composition comprising *Leuconostoc* bacteria-derived vesicles as an active ingredient to a subject in need thereof.

In addition, the present invention provides a use of a composition comprising *Leuconostoc* bacteria-derived vesicles as an active ingredient for preventing or treating inflammatory diseases or cancer.

In addition, the present invention provides a use of *Leuconostoc* bacteria-derived vesicles for preparing a drug for treating inflammatory diseases or cancer.

### [Advantageous Effects]

As it was confirmed that the secretion of inflammatory mediators was inhibited when the *Leuconostoc* bacteria-derived vesicles according to the present invention were administered to inflammatory cells, and that cancer development was significantly inhibited when the vesicles were orally administered to cancer animal models, it is expected that the *Leuconostoc* bacteria-derived vesicles can be widely used as a therapeutic agent for the alleviation, prevention, or treatment of inflammatory diseases or cancer.

### [Description of Drawings]

FIG. 1 is a view illustrating the result of confirming an anti-inflammatory effect of *Leuconostoc* bacteria-derived vesicles according to one embodiment of the present invention;
FIG. 2 is a view illustrating an experimental protocol for evaluating the cancer prevention efficacy of *Leuconostoc* bacteria-derived vesicles according to one embodiment of the present invention;
FIG. 3 is a view illustrating the result of measuring a cancer size over time after oral administration of *Leuconostoc* bacteria-derived vesicles to a cancer model prepared by implanting cancer cells to confirm the cancer prevention efficacy of the *Leuconostoc* bacteria-derived vesicles according to one embodiment of the present invention;
FIG. 4A is a view illustrating an experimental protocol for evaluating the cancer treatment efficacy of *Leuconostoc* bacteria-derived vesicles according to one embodiment of the present invention; and
FIG. 4B is a view illustrating a cancer size over time after intraperitoneal administration of *Leuconostoc* bacteria-derived vesicles to a cancer model prepared by implanting cancer cells to evaluate the cancer treatment efficacy of the *Leuconostoc* bacteria-derived vesicles according to one embodiment of the present invention.

### [Best Mode]

In the present invention, it was confirmed that *Leuconostoc* bacteria-derived vesicles effectively inhibit the secretion of IL-6, which is a representative mediator inducing inflammatory diseases, thereby exhibiting a significant therapeutic effect on inflammatory diseases.

In addition, in the present invention, it was confirmed from the result of evaluating a cancer inhibitory effect by orally administering *Lactobacillus plantarum-*derived vesicles, *Lactobacillus rhamnosus-derived* vesicles, *Lactococcus lactis-*derived vesicles, and *Leuconostoc* bacteria-derived vesicles that only *Leuconostoc* bacteria-derived vesicles effectively inhibit cancer development such that the *Leuconostoc* bacteria-derived vesicles exhibit a significant cancer-preventing effect. In addition, as a result of evaluating cancer treatment efficacy by intraperitoneally administering the *Leuconostoc* bacteria-derived vesicles after cancer development, it was confirmed that the *Leuconostoc* bacteria-derived vesicles exhibit a significant cancer treatment effect.

Accordingly, the present invention relates to a composition for alleviating, preventing or treating inflammatory diseases or cancer, including *Leuconostoc* bacteria-derived vesicles as an active ingredient.

Hereinafter, the present invention will be described in detail.

The present invention provides a pharmaceutical composition for preventing or treating inflammatory diseases or cancer, comprising *Leuconostoc* bacteria-derived vesicles as an active ingredient.

As used herein, "extracellular vesicle" or "vesicle" refers to a structure formed of a nano-sized membrane secreted from various bacteria, and includes, for example, a vesicle derived from gram-negative bacteria such as *E. coli*, which has, an endotoxin (lipopolysaccharide), a toxic protein, and both bacterial DNA and RNA, or a vesicle derived from gram-positive bacteria such as bacteria of the genus *Micrococcus*, which have outer membrane vesicles (OMVs), a protein and a nucleic acid as well as components of a bacterial cell wall, such as peptidoglycan and lipoteichoic acid.

In the present invention, the vesicles encompass all membrane structures that are naturally secreted from *Leuconostoc* bacteria or artificially produced. The vesicles may be isolated from a culture medium containing *Leuconostoc* bacterial cells using one or more methods selected from the group consisting of centrifugation, ultracentrifugation, high pressure treatment, extrusion, sonication, cell lysis, homogenization, freeze-thawing, electroporation, mechanical degradation, chemical treatment, filtration with a filter, gel filtration chromatography, free-flow electrophoresis, and capillary electrophoresis. In addition, the present invention may further include washing for removing impurities and concentration of the collected vesicles.

In the method of isolating vesicles from the culture medium or fermented food of the *Leuconostoc* bacteria, the culture solution or fermented food is not particularly limited as long as it includes vesicles. For example, the vesicles may be isolated using a method such as centrifugation, ultracentrifugation, filtration with a filter, gel filtration chromatography, free-flow electrophoresis, or capillary electrophoresis, and a combination thereof, and may further include washing for removing impurities and concentration of the collected vesicles.

The vesicles of the present invention may be isolated from the culture medium of *Leuconostoc* bacteria or food that has been prepared by adding *Leuconostoc* bacteria, and may be naturally secreted from *Leuconostoc* bacteria or artificially produced, but the present invention is not limited thereto.

The vesicles isolated by the above method in the present invention may have an average diameter of 10 to 1000 nm, 10 to 900 nm, 10 to 800 nm, 10 to 700 nm, 10 to 600 nm, 10 to 500 nm, 10 to 400 nm, 10 to 300 nm, 10 to 220 nm, 10 to 200 nm, 10 to 100 nm, 10 to 90 nm, 10 to 80 nm, 10 to 70 nm, 10 to 60 nm, 10 to 50 nm, 10 to 40 nm, or 20 to 40 nm, but the present invention is not limited thereto.

The expression "comprised as an active ingredient" means containing a sufficient amount to achieve the efficacy or activity of the *Leuconostoc* bacteria-derived vesicles.

In the specification, "inflammatory disease" means a disease induced by an inflammatory response in the body, and the inflammatory disease may be, but is not limited to, one or more respiratory inflammatory diseases selected from the group consisting of asthma, chronic obstructive pulmonary disease, pneumonia, interstitial pneumonia, idiopathic pulmonary fibrosis, and rhinitis;
one or more skin inflammatory diseases selected from the group consisting of atopic dermatitis, psoriasis, acne, contact dermatitis, and hair loss;
one or more digestive inflammatory diseases selected from the group consisting of gastritis, digestive ulcers, acute enteritis, chronic enteritis, Crohn's disease, inflammatory bowel disease, Behcet's colitis, ulcerative colitis, bacterial colitis, alcoholic steatohepatitis, non-alcoholic steatohepatitis, chronic hepatitis, pancreatitis, and cholangitis;
vaginitis; and
one or more bone and joint inflammatory diseases selected from the group consisting of osteoarthritis, degenerative arthritis, and rheumatoid arthritis, and may include any disease mediated by IL-6.

In the specification, "cancer" includes diseases that occur by transforming normal cells into cancer cells, characterized by abnormal proliferation, due to repetitive stress, and in the present invention, the cancer may be, but is not limited to, one or more selected from the group consisting of colorectal cancer, stomach cancer, lung cancer, liver cancer, biliary tract cancer, pancreatic cancer, breast cancer, ovarian cancer, kidney cancer, bladder cancer, prostate cancer, blood cancer, head and neck cancer, colon cancer, bone marrow cancer, uterine cancer, melanoma, brain cancer, thyroid cancer, and lymphoma, and may include any malignant disorder.

The amount of the vesicles in the composition of the present invention may be appropriately adjusted depending on the symptoms of a disease, the degree of progression of symptoms, the condition of a patient, and the like, and may range from, for example, 0.0001 wt% to 99.9 wt% or 0.001 wt% to 50 wt% with respect to a total weight of the composition, but the present invention is not limited thereto. The amount ratio is a value based on the amount of dried product from which a solvent is removed.

In the present invention, "pharmaceutical composition" is prepared to prevent or treat a disease, and may be formulated and used in various forms according to conventional methods. For example, the pharmaceutical composition may be formulated into an oral form such as a powder, a granule, a tablet, a capsule, a suspension, an emulsion, or a syrup, or in formula of external use, a suppository and a sterile injectable solution.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and *Primojel*^{®}; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Non-limiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields. In addition, the pharmaceutical composition according to the present invention may be administered daily at 0.0001 to 50 mg/kg or 0.001 to 50 mg/kg. The administration may be conducted daily at once or in several divided portions. The above dose does not limit the scope of the present invention in any way.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined by the type of drug, which is an active ingredient, along with several related factors such as a disease to be treated, an administration route, a patient's age, sex and weight, and the severity of the disease.

In addition, the present invention provides a method for preventing or treating inflammatory diseases or cancer, the method comprising administering a composition comprising *Leuconostoc* bacteria-derived vesicles as an active ingredient to a subject in need thereof.

In addition, the present invention provides a use of a composition comprising *Leuconostoc* bacteria-derived vesicles as an active ingredient for preventing or treating inflammatory diseases or cancer.

In addition, the present invention provides a use of *Leuconostoc* bacteria-derived vesicles for preparing a drug for treating inflammatory diseases or cancer.

As used herein, the "subject" refers to a subject in need of treatment of a disease, and more specifically, refers to a mammal such as a human or a non-human primate, a mouse, a rat, a dog, a cat, a horse, and a cow.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "alleviation" as used herein means all actions that reduce the degree of parameters related to a target disease, e.g., symptoms via administration of the composition according to the present invention.

In addition, the present invention provides a food composition for preventing or alleviating inflammatory diseases or cancer, comprising *Leuconostoc* bacteria-derived vesicles as an active ingredient.

The food composition may be a health functional food composition, but is not limited thereto.

The vesicles according to the present invention may be used by adding the vesicles as is to food or may be used together with other foods or food ingredients, but may be appropriately used according to a typical method. The mixed amount of the active ingredient may be suitably determined depending on the purpose of use thereof (for prevention or alleviation). In general, when a food or beverage is prepared, the composition of the present invention is added in an amount of 15 wt% or less, preferably 10 wt% or less based on the raw materials. However, for long-term intake for the purpose of health and hygiene or for the purpose of health control, the amount may be less than the above-mentioned range, and the vesicles have no problem in terms of stability, so the active ingredient may be used in an amount more than the above-mentioned range.

The type of food is not particularly limited. Examples of food to which the material may be added include meats, sausage, bread, chocolate, candies, snacks, confectioneries, pizza, instant noodles, other noodles, gums, dairy products including ice creams, various soups, beverages, tea, drinks, alcoholic beverages, vitamin complexes, and the like, and include all health functional foods in a typical sense.

The health beverage composition according to the present invention may contain various flavors or natural carbohydrates, and the like as additional ingredients as in a typical beverage. The above-described natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, it is possible to use a natural sweetener such as thaumatin and stevia extract, a synthetic sweetener such as saccharin and aspartame, and the like. The proportion of the natural carbohydrates is generally about 0.01 to 0.20 g, or about 0.04 to 0.10 g per 100 ml of the composition of the present invention.

In addition to the aforementioned ingredients, the composition of the present invention may contain various nutrients, vitamins, electrolytes, flavors, colorants, pectic acids and salts thereof, alginic acid and salts thereof, organic acids, protective colloid thickeners, pH adjusters, stabilizers, preservatives, glycerin, alcohols, carbonating agents used in carbonated drinks, and the like. In addition, the composition of the present invention may contain flesh for preparing natural fruit juice, fruit juice drinks, and vegetable drinks. These ingredients may be used either alone or in combinations thereof. The proportion of these additives is not significantly important, but is generally selected within a range of 0.01 to 0.20 part by weight per 100 parts by weight of the composition of the present invention.

### [Modes of the Invention]

Hereinafter, preferred Examples for helping the understanding of the present invention will be suggested. However, the following Examples are provided only to more easily understand the present invention, and the contents of the present invention are not limited by the following Examples.

### [Examples]

### Example 1: Anti-inflammatory effects of Leuconostoc bacteria and bacteria-derived vesicles

To isolate *Leuconostoc* bacterial cells and bacteria-derived vesicles, first, representative *Leuconostoc* bacteria, such as *Leuconostoc mesenteroides,* were inoculated into a de Man-Rogosa and Sharpe (MRS) medium and incubated at 37 °C and 200 rpm until the absorbance (OD₆₀₀ₙₘ) reached 1.0 to 1.5, and then reinoculated into a Luria Bertani (LB) medium and incubated. In addition, the culture medium containing the bacterial cells was collected and centrifuged at 10,000 g and 4 °C for 20 minutes to obtain bacterial cells and a supernatant from which the bacterial cells were removed. The bacterial cells were heated at 70 °C for 10 minutes to prepare a lysate to be used in the experiment. To isolate vesicles from the supernatant, the collected supernatant was filtered again using a 0.22-µm filter, and the filtered supernatant was concentrated to a volume of 50 mL or less using a 100 kDa Pellicon 2 Cassette filter membrane (Merck Millipore) and a MasterFlex pump system (Cole-Parmer). The concentrated supernatant was filtered again using a 0.22-µm filter to isolate *Leuconostoc* bacteria-derived vesicles. The amounts of proteins included in the bacterial cells and the supernatant were measured using a Pierce BCA protein assay kit (Thermo Fisher Scientific).

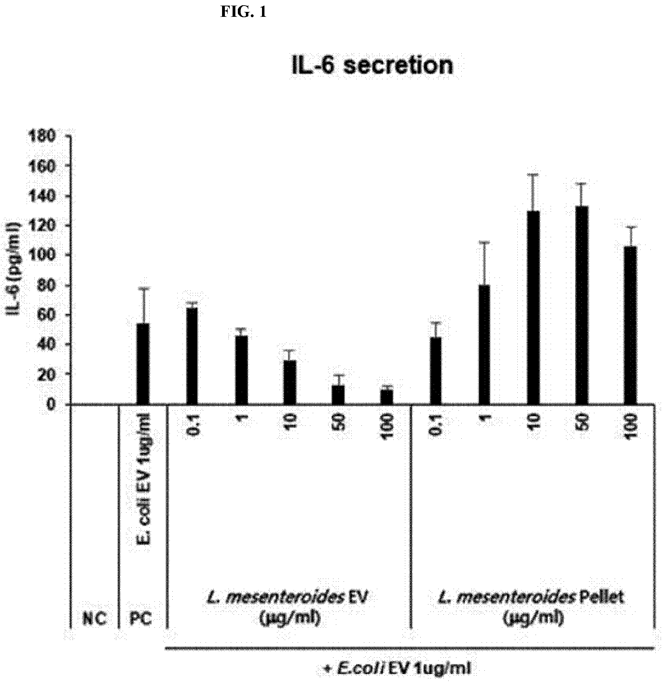

To evaluate the anti-inflammatory efficacy of the *Leuconostoc* bacterial cells and the bacteria-derived vesicles, a mouse macrophage cell line Raw 264.7 was pretreated with either the *Leuconostoc mesenteroides* cells or the bacteria-derived vesicles at 0.1, 1, 10, 50, or 100 µg/mL and incubated, and then a secretion level of IL-6 was measured. In further detail, the Raw 264.7 cells were seeded at 5×10 cells/well in a 48-well plate, treated with *Leuconostoc mesenteroides*-derived vesicles or *Leuconostoc mesenteroides* cells, which were diluted with a serum-free DMEM medium, and then incubated for 12 hours. Then, after treating E. *coli*-derived vesicles, which is a factor inducing inflammation, at 1 µg/mL, the cells were further incubated for 12 hours. Then, the secretion level of IL-6 was measured. As a result, as shown in FIG. 1, while the vesicles inhibited IL-6 secretion in a dose-dependent manner, the bacterial cells tended to increase IL-6 secretion in a dose-dependent manner.

### Example 2: Isolation of vesicles from bacteria culture medium of Lactobacillus plantarum. Lactobacillus rhamnosus, and Lactococcus lacti

To isolate vesicles derived from lactic acid bacteria, such as *Lactobacillus plantarum*, *Lactobacillus rhamnosus*, and *Lactococcus lactis*, the bacteria were inoculated into an MRS medium and incubated at 37 °C and 200 rpm until the absorbance (OD₆₀₀ₙₘ) reached 1.0 to 1.5, and then reinoculated into a LB medium and incubated. Then, the culture medium containing the bacterial cells was recovered and centrifuged at 10,000 g and 4 °C for 20 minutes to obtain a supernatant from which the bacterial cells were removed. The obtained supernatant was filtered again using a 0.22-µm filter, the filtered supernatant was concentrated to a volume of 50 mL or less using a 100 kDa Pellicon 2 Cassette filter membrane (Merck Millipore) and a MasterFlex pump system (Cole-Parmer). The concentrated supernatant was filtered again using a 0.22-µm filter to isolate bacteria-derived vesicles. The amount of proteins contained in the supernatant was measured using a Pierce BCA protein assay kit (Thermo Fisher Scientific).

### Example 3: Experimental protocol for evaluating anti-cancer efficacy of vesicles derived from lactic acid bacteria such as Lactobacillus plantarum. Lactobacillus rhamnosus, Lactococcus lactis, and Leuconostoc mesenteroides

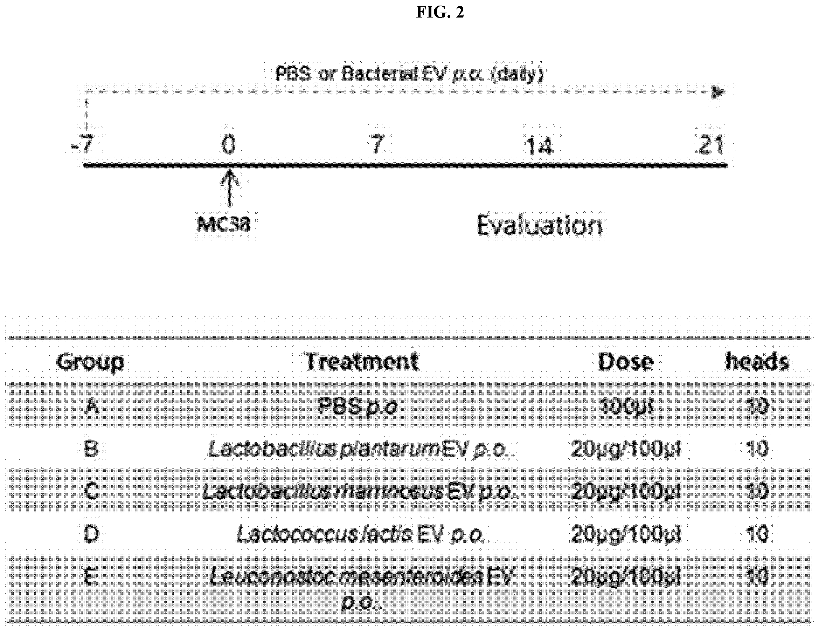

To confirm the anti-cancer effects of vesicles derived from lactic acid bacteria, such as *Lactobacillus plantarum*, *Lactobacillus rhamnosus*, *Lactococcus lactis*, and *Leuconostoc mesenteroides,* as shown in FIG. 2, MC38 cells, which are mouse cancer cells, were subcutaneously injected into mice to generate cancer. In further detail, after 6-week-old male C57BL/6 mice were raised under general conditions for 3 hours and acclimated, 20 µg of the vesicles derived from bacteria, such as *Lactobacillus plantarum*, *Lactobacillus rhamnosus*, *Lactococcus lactis*, and *Leuconostoc mesenteroides*, were each orally administered for 1 week. As a control (PBS), PBS was orally administered for 1 week. On day 8 after the start of the experiment, 1 × 10⁶ MC38 cells, which are mouse cancer cells, were subcutaneously injected into the mice. Then, 20 µg of PBS, or each of *Lactobacillus plantarum-*, *Lactobacillus rhamnosus-*, *Lactococcus lactis-*, and *Leuconostoc mesenteroides*-derived vesicles were orally administered for 5 days a week, once a day, for 3 weeks, and a cancer size was measured.

### Example 4: Cancer prevention efficacy of Lactobacillus plantarum-. Lactobacillus rhamnosus-, Lactococcus lactis-, and Leuconostoc mesenteroides-derived vesicles

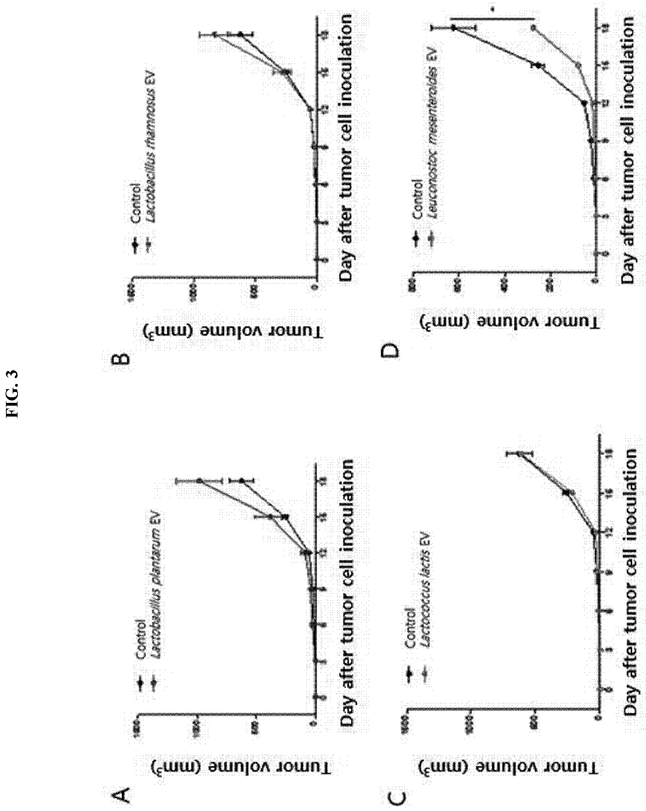

By the method of Example 3, the cancer prevention efficacy of vesicles derived from lactic acid bacteria, such as *Lactobacillus plantarum*, *Lactobacillus rhamnosus*, *Lactococcus lactis*, and *Leuconostoc mesenteroides,* was evaluated. As a result, as shown in FIGS. 3A to 3C, in the control administered only PBS, the cancer size exponentially increased, and in the experimental groups orally administered *Lactobacillus plantarum-derived* vesicles, *Lactobacillus rhamnosus*-derived vesicles, and *Lactococcus lactis-*derived vesicles, the cancer sizes were not significantly reduced compared to the control. On the other hand, as shown in FIG. 3D, in the mouse orally administered the *Leuconostoc mesenteroides-*derived vesicles, it was confirmed that the cancer size was significantly reduced compared to the control and became approximately 50% smaller. From the above results, it was able to be confirmed that the *Leuconostoc* bacteria-derived vesicles can effectively inhibit cancer growth.

### Example 5: Cancer treatment efficacy of Leuconostoc mesenteroides-derived vesicles

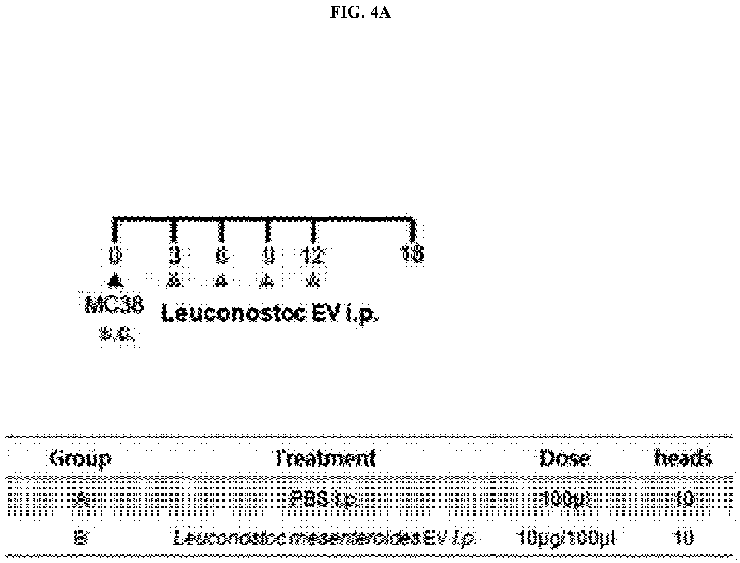

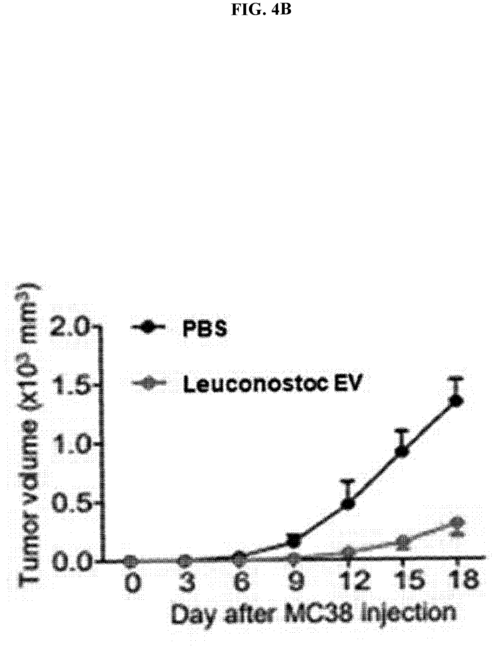

The cancer treatment efficacy of *Leuconostoc mesenteroides*-derived vesicles in a cancer mouse model was evaluated. As shown in FIG. 4A, MC38 cells, which are mouse cancer cells, were subcutaneously injected into the mouse to generate cancer. On day 3, 6, 9, and 12 after the administration of cancer cells, 10 µg of *Leuconostoc mesenteroides*-derived vesicles was intraperitoneally administered, and as a control, PBS was intraperitoneally administered. As a result, as shown in FIG. 4B, in the control administered only PBS, the cancer size exponentially increased, and in the mice intraperitoneally administered the *Leuconostoc mesenteroides*-derived vesicles, the cancer size was significantly reduced compared to the control. From the above results, it was able to be confirmed that the *Leuconostoc* bacteria-derived vesicles can effectively treat cancer.

From the above results, since it was confirmed that the *Leuconostoc* bacteria-derived vesicles of the present invention exhibit a preventive and/or treatment effect on inflammatory diseases and cancer by not only inhibiting the secretion of an inflammatory mediator but also effectively inhibiting cancer growth, it is expected that the *Leuconostoc* bacteria-derived vesicles of the present invention can be used for alleviating, preventing, or treating inflammatory diseases or cancer.

It was confirmed that, when *Leuconostoc* bacteria-derived vesicles according to the present invention were administered to inflammatory cells, the secretion of an inflammatory mediator was inhibited, and when the vesicles were administered to a cancer animal model, cancer development was significantly suppressed. Therefore, the *Leuconostoc* bacteria-derived vesicles are expected to be widely used as a drug for alleviating, preventing, or treating inflammatory diseases or cancer.

The above-described description of the present invention is merely provided to exemplify the present invention, and it will be understood by those of ordinary skill in the art to which the present invention belongs that the present invention can be implemented in modified forms without departing from the essential features of the present invention. Therefore, the exemplary embodiments described above should be interpreted as illustrative and not limited in any aspect.

### [Industrial Applicability]

When *Leuconostoc* bacteria-derived vesicles according to the present invention were administered to inflammatory cells, the secretion of an inflammatory mediator was inhibited, and when the vesicles were orally administered to a cancer animal model, it was confirmed that cancer development was significantly inhibited. Therefore, *Leuconostoc* bacteria-derived vesicles have industrially applicability because they can be widely used as a drug for alleviating, preventing, or treating inflammatory diseases or cancer.

## Claims

1. A pharmaceutical composition for preventing or treating inflammatory diseases or cancer, comprising *Leuconostoc* bacteria-derived vesicles as an active ingredient.

2. The pharmaceutical composition of claim 1, wherein the inflammatory disease is one or more selected from the group consisting of the following diseases:
one or more respiratory inflammatory diseases selected from the group consisting of asthma, chronic obstructive pulmonary disease, pneumonia, interstitial pneumonia, idiopathic pulmonary fibrosis, and rhinitis;
one or more skin inflammatory diseases selected from the group consisting of atopic dermatitis, psoriasis, acne, contact dermatitis, and hair loss;
one or more digestive inflammatory diseases selected from the group consisting of gastritis, digestive ulcers, acute enteritis, chronic enteritis, Crohn's disease, inflammatory bowel disease, Behcet's colitis, ulcerative colitis, bacterial colitis, alcoholic steatohepatitis, non-alcoholic steatohepatitis, chronic hepatitis, pancreatitis, and cholangitis;
vaginitis; and
one or more bone and joint inflammatory diseases selected from the group consisting of osteoarthritis, degenerative arthritis, and rheumatoid arthritis.

3. The pharmaceutical composition of claim 1, wherein the cancer is one or more selected from the group consisting of colon cancer, stomach cancer, lung cancer, liver cancer, biliary tract cancer, pancreatic cancer, breast cancer, ovarian cancer, kidney cancer, bladder cancer, prostate cancer, blood cancer, head and neck cancer, colon-rectal cancer, bone marrow cancer, uterine cancer, melanoma, brain cancer, thyroid cancer, and lymphoma.

4. The pharmaceutical composition of claim 1, wherein the the *Leuconostoc* bacteria is one or more selected from the group consisting of *Leuconostoc carnosum*, *Leuconostoc citreum*, *Leuconostoc fallax*, *Leuconostoc falkenbergense*, *Leuconostoc ficulneum*, *Leuconostoc fructosum*, *Leuconostoc garlicum*, *Leuconostoc gasicomitatum*, *Leuconostoc gelidum*, *Leuconostoc inhae*, *Leuconostoc kimchii*, *Leuconostoc lactis*, *Leuconostoc mesenteroides*, *Leuconostoc miyukkimchii*, *Leuconostoc palmae*, *Leuconostoc pseudoficulneum*, *Leuconostoc pseudomesenteroides*, *Leuconostoc rapi*, and *Leuconostoc suionicum.*

5. The pharmaceutical composition of claim 1, wherein the vesicles have an average diameter of 10 to 300 nm.

6. The pharmaceutical composition of claim 1, wherein the vesicles are naturally secreted or artificially produced from *Leuconostoc* bacteria.

7. The pharmaceutical composition of claim 1, the vesicles are isolated from the culture medium of *Leuconostoc* bacteria or food that has been prepared by adding *Leuconostoc* bacteria.

8. A food composition for preventing or alleviating inflammatory diseases or cancer, comprising *Leuconostoc* bacteria-derived vesicles as an active ingredient.

9. The food composition of claim 8, wherein the inflammatory disease is one or more selected from the group consisting of the following diseases:
one or more respiratory inflammatory diseases selected from the group consisting of asthma, chronic obstructive pulmonary disease, pneumonia, interstitial pneumonia, idiopathic pulmonary fibrosis, and rhinitis;
one or more skin inflammatory diseases selected from the group consisting of atopic dermatitis, psoriasis, acne, contact dermatitis, and hair loss;
one or more digestive inflammatory diseases selected from the group consisting of gastritis, digestive ulcers, acute enteritis, chronic enteritis, Crohn's disease, inflammatory bowel disease, Behcet's colitis, ulcerative colitis, bacterial colitis, alcoholic steatohepatitis, non-alcoholic steatohepatitis, chronic hepatitis, pancreatitis, and cholangitis;
vaginitis; and
one or more bone and joint inflammatory diseases selected from the group consisting of osteoarthritis, degenerative arthritis, and rheumatoid arthritis.

10. The food composition of claim 8, wherein the cancer is one or more selected from the group consisting of colon cancer, stomach cancer, lung cancer, liver cancer, biliary tract cancer, pancreatic cancer, breast cancer, ovarian cancer, kidney cancer, bladder cancer, prostate cancer, blood cancer, head and neck cancer, colon-rectal cancer, bone marrow cancer, uterine cancer, melanoma, brain cancer, thyroid cancer, and lymphoma.

11. The food composition of claim 8, wherein the the *Leuconostoc* bacteria is one or more selected from the group consisting of *Leuconostoc carnosum*, *Leuconostoc citreum, Leuconostoc fallax*, *Leuconostoc falkenbergense*, *Leuconostoc ficulneum*, *Leuconostoc fructosum*, *Leuconostoc garlicum*, *Leuconostoc gasicomitatum*, *Leuconostoc gelidum*, *Leuconostoc inhae*, *Leuconostoc kimchii*, *Leuconostoc lactis*, *Leuconostoc mesenteroides*, *Leuconostoc miyukkimchii*, *Leuconostoc palmae*, *Leuconostoc pseudoficulneum*, *Leuconostoc pseudomesenteroides*, *Leuconostoc rapi*, and *Leuconostoc suionicum.*

12. The food composition of claim 8, wherein the vesicles are naturally secreted or artificially produced from *Leuconostoc* bacteria.

13. A method for preventing or treating inflammatory diseases or cancer, the method comprising administering a composition comprising *Leuconostoc* bacteria-derived vesicles as an active ingredient to a subject in need thereof.

14. A use of a composition comprising *Leuconostoc* bacteria-derived vesicles as an active ingredient for preventing or treating inflammatory diseases or cancer.

15. A use of *Leuconostoc* bacteria-derived vesicles for preparing a drug for treating inflammatory diseases or cancer.
